# FASCICULE DE BREVET EUROPEEN

(11) **EP 0 682 936 B1**
(45) Date de publication et mention de la délivrance du brevet: **14.01.1998**
(21) Numéro de dépôt: 95400610.2
(22) Date de dépôt: 20.03.1995
(51) Int. Cl.: A61K 7/06

(54) **Utilisation d'une gomme de guar non ionique ou anionique dans un procédé de mise en forme non permanente des fibres kératiniques**
Verwendung von eine nichtionische oder anionische Guargummi in einem nicht dauerhaften Verformen von keratischen Fasern
Use of a non ionic or anionic guar gum in a non permanent shaping process of keratinic fibres

(30) Priorité: 25.04.1994 FR 9404961; 20.01.1995 FR 9500658
(43) Date de publication de la demande: 22.11.1995
(73) Titulaire: L'OREAL, 75008 Paris (FR)
(72) Inventeur: Samain, Henri, F-91570 Bievres (FR); Cretois, Isabelle, F-92110 Clichy (FR)
(74) Mandataire: Tezier Herman, Béatrice

(56) Documents cités:
- EP-A- 0 018 717
- DE-A- 3 632 030
- FR-A- 2 067 649
- US-A- 4 330 438
- US-A- 4 472 297
- SEIFEN ÖLE FETTE WACHSE, vol. 110, no. 17, Octobre 1984 AUGSBURG, DEUTSCHLAND, pages 509-512, BUSCH P. ET AL 'GUAR-HYDROXYPROPYLTRIMETHYLAMMONIUM ALS HAARKONDITIONIERENDER WIRKSTOFF'
- CHEMICAL ABSTRACTS, vol. 120, no. 18, 2 Mai 1994 Columbus, Ohio, US; abstract no. 226526, IKEDA YOSHIO ET AL 'Hair setting aerosols containing film-forming polymers and guar gums'
- COSMET. TOILETRIES, vol. 99, no. 6, 1984 pages 83-87, FREELAND M. ET AL 'Cationic guar gum '

## Description

La présente invention a pour objet l'utilisation d'une gomme de guar non ionique au anionique en tant que seul agent de maintien et/ou de mise en forme dans des compositions cosmétiques destinées à une mise en forme non permanente des fibres kératiniques, et un procédé de déformation et/ou de mise en forme non permanente de ces dernières, en particulier sous la forme d'une mise en plis, utilisant ce composé.

On sait que l'on désigne en coiffure sous le terme de "mise en plis" l'opération simple qui consiste à donner aux cheveux une forme non définitive et temporaire (généralement ondulée telle que boucles, frisures et autres) qui disparaît instantanément lorsque les cheveux sont à nouveau mouillés, en particulier lorsque ceux-ci sont soumis à l'action de lavages à l'eau ou par shampooings, et ceci par opposition à une opération de déformation dite permanente, au cours de laquelle de véritables traitements et/ou transformations chimiques (réduction/oxydation) doivent être mis en oeuvre sur les fibres kératiniques, la forme finale imposée aux cheveux ne devenant dans ce cas plus du tout (ou que très peu) sensible aux agents extérieurs susmentionnés.

La technique la plus usuelle pour réaliser une mise en plis (ou déformation non permanente) des cheveux consiste à mettre tout d'abord sous tension (avec des supports classiques de type bigoudis, rouleaux de mise en plis et autres) des cheveux préalablement mouillés ou encore humides, puis à sécher les cheveux ainsi mis sous tension sous un casque de coiffure chauffant à une température comprise entre 30°C et 60°C pendant un temps qui peut varier de 20 à 60 mn selon la masse des cheveux à sécher, à ensuite retirer des cheveux ainsi séchés les moyens de mises sous tension utilisés précédemment et enfin à donner un coup de peigne de finition pour obtenir la coiffure avec la forme finale recherchée.

On peut également réaliser un brushing qui consiste à mettre en forme les cheveux à l'aide d'une brosse pendant le séchage de ces derniers.

L'inconvénient principal attaché à ces techniques est qu'elles nécessitent toujours l'emploi d'un matériel de soutien (rouleaux, brosses, pinces et autres) pendant le séchage des cheveux, car tant que ces derniers ne sont pas secs, ils ne peuvent tenir seuls dans la forme désirée. Ces techniques présentent néanmoins pour avantage, en contrepartie, de conserver aux cheveux un toucher naturel et agréable.

Des compositions de fixation capillaires en aérosol sont décrites dans les résumés de la demande JP-A-5 345708, elles contiennent un dérivé de gomme de guar non ionique ou cationique et un polymère filmogène.

La demande FR 2.067.649 décrit des compositions capillaires défrisantes contenant des galactomannanes. L'article de M. S. Freeland (Cosmetics & Toiletries, 99(6), p. 83-87) décrit les propriétés des gommes de guar cationiques. Le brevet US 4,330,438 décrit des shampooings concentrés en poudre contenant un tensioactif anionique et un dérivé non ionique de galactomannane.

Pour effectuer la mise en forme des cheveux sans matériel de soutien, on a déjà proposé l'utilisation de compositions sous forme de gel qui sont généralement à base de polymères acryliques réticulés. Ces compositions présentent toutefois l'inconvénient de laisser un dépôt indésirable sur les cheveux qui nuit aux propriétés cosmétiques de ces derniers : ainsi, en fin d'opération, les cheveux sont peu brillants, rêches ou collants.

La présente invention a notamment pour but de résoudre les problèmes ci-dessus. Plus précisément encore, la présente invention vise à proposer un procédé de mise en forme non permanente des fibres kératiniques qui, tout en permettant de s'affranchir de l'emploi des habituels matériels de soutien, soit néanmoins capable de conduire, en fin d'opération, à des fibres kératiniques présentant un toucher et un aspect visuel neutres.

Or, à la suite d'importantes recherches menées sur la question, il a maintenant été trouvé par la Demanderesse, et ceci de façon tout à fait inattendue et surprenante, que ce but, et d'autres, pouvaient être atteints en mettant, en oeuvre, dans un support cosmétiquement acceptable, une gomme de guar non ionique ou anionique qui est présente dans ledit support comme seul agent de mise en forme et/ou de maintien des fibres kératiniques.

Cette découverte est à la base de la présente invention.

La présente invention a ainsi pour objet l'utilisation, dans des compositions cosmétiques destinées à une mise en forme non permanente des fibres kératiniques, d'une gomme de guar non ionique au anionique en tant que seul agent de mise en forme et/ou de maintien desdites fibres kératiniques, en particulier les cheveux, les cils ou les sourcils.

La présente invention a également pour objet un procédé de déformation et/ou de mise en forme non permanente des fibres kératiniques, qui est caractérisé par le fait qu'il comprend les étapes suivantes :
(i) on applique sur les fibres kératiniques sèches ou humides une composition cosmétique contenant, à titre d'unique agent de mise en forme et/ou de maintien, une gomme de guar non ionique ou anionique, ladite gomme étant présente dans un support cosmétiquement acceptable plus particulièrement de type aqueux ou hydroalcoolique,
(ii) on donne une forme aux fibres kératiniques à l'aide des doigts, d'un peigne ou d'une brosse,
(iii) on procède au séchage des fibres kératiniques ou on les laisse sécher,
(iv) et éventuellement, on coiffe les fibres kératiniques avec un peigne, une brosse ou avec les mains.

Ce procédé s'applique plus particulièrement aux cheveux.

Selon l'invention, on peut utiliser les gommes de guar non ioniques chimiquement modifiées ou non modifiées.

Les gommes de guar non ioniques non modifiées sont par exemple les produits vendus sous la dénomination VIDOGUM® GH 175 par la société UNIPECTINE et sous la dénomination JAGUAR® C par la société MEYHALL.

Les gommes de guar non-ioniques modifiées utilisables selon l'invention sont de préférence modifiées par des groupements hydroxyalkyle en C₁-C₆.

Parmi les groupements hydroxyalkyle, on peut mentionner à titre d'exemple, les groupements hydroxyméthyle, hydroxyéthyle, hydroxypropyle et hydroxybutyle.

Ces gommes de guar non ioniques sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

Le taux d'hydroxyalkylation, qui correspond au nombre de molécules d'oxyde d'alkylène consommées par le nombre de fonctions hydroxy'le libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8®, JAGUAR HP60® et JAGUAR HP120®, JAGUAR DC 293® et JAGUAR HP 105® par la société MEYHALL, ou sous la dénomination GALACTASOL 4H4FD2® par la société AQUALON.

Les gommes de guar non-ioniques peuvent également être modifiées par des groupements alkyle ou hydroxyakyle en C₁-C₆ et par des chaînes grasses ayant de 8 à 30 atomes de carbone telles que les groupements alkyle ou alkényle, linéaires ou ramifiés.

De tels composés sont par exemple décrits dans les demandes de brevets EP 0 323 627 (US 4960876) et EP 0 281 360.

Les gommes de guar anioniques utilisables selon l'invention peuvent être modifiées par des groupements anioniques tels que carboxyliques ou phosphates et de préférence par des groupements carboxyméthyle.

En plus des groupements anioniques, les gommes de guar peuvent posséder des groupements hydroxyalkyle en C₁-C₆. Parmi les groupements hydroxyalkyle, on peut mentionner à titre d'exemple, les groupements hydroxyméthyle, hydroxyéthyle, hydroxypropyle et hydroxybutyle.

De telles gommes de guar anioniques éventuellement modifiées par des groupements hydroxyalkyle sont par exemple vendues sous les dénominations commerciales GALACTASOL 60 H 3 FD® et CARBOXYMETHYL GUAR® par la société AQUALON.

De préférence, on utilise une gomme de guar non ionique et, parmi ces gommes de guar non ioniques, plus particulièrement les gommes de guar modifiées par des groupement hydroxyalkyles.

La gomme de guar est généralement présente dans la composition cosmétique en une quantité comprise entre 0,2 et 5% en poids par rapport au poids total de la composition, et de préférence entre 0,6 et 2% en poids.

Le support cosmétiquement acceptable contient de préférence de l'eau et/ou un mélange d'eau et de solvants cosmétiquement acceptables qui sont notamment choisis parmi les alcools inférieurs tels que l'éthanol ou l'isopropanol.

Les solvants peuvent être présents dans des concentrations généralement comprises entre 0 et 50% en poids par rapport au poids total de la composition.

Le pH des compositions utilisées selon l'invention est généralement compris entre 2 et 12.

La composition peut enfin contenir en outre des additifs usuels choisis parmi les conservateurs, les agents de régulation de pH, les parfums. Bien entendu, l'homme de l'art veillera à choisir les éventuels additifs de manière telle que les propriétés de maintien de la composition ne soient pas, ou substantiellement pas altérées par ces additifs.

Une composition conforme à l'invention est de préférence de type non moussante.

Les compositions utilisées selon l'invention sont par exemple des compositions capillaires de fixation et/ou de coiffage, des compositions de maquillage des cils ou des sourcils telles que les mascaras.

### EXEMPLE 1

On a préparé un gel (1), conforme à l'invention, de composition suivante :

| | |
|---|---|
| - Gomme de guar non-ionique modifiée par des groupements hydroxypropyle vendue sous la dénomination commerciale JAGUAR HP60® par la société MEYHALL | 1,7 g |
| - Eau qsp | 100 g |

On a préparé un gel (2), comparatif, de composition suivante :

| | |
|---|---|
| - Polymère d'acide acrylique réticulé vendu sous la dénomination commerciale CARBOPOL 934® par la société GOODRICH | 1,7 g |
| - Monoéthanolamine qs | pH 7 |
| - Eau déminéralisée q.s.p. | 100 g |

Sur des cheveux lavés et essorés, on a appliqué par demi-tête 10 g du gel (1) à gauche et 10 g du gel (2) à droite. On a formé des boucles en enroulant les cheveux sur eux-mêmes à l'aide des doigts sur les deux parties de la tête. On a ensuite séché les cheveux sous un casque et lorsque les cheveux étaient secs, on a peigné les cheveux.

Du côté gauche (invention), le démêlage est facile, la coiffure est gonflante. Les cheveux sont doux et brillants.

Du côté droit (comparatif), le démêlage est au contraire difficile, les cheveux sont rêches et ils ne sont pas brillants.

### EXEMPLE 2

On a préparé un autre gel conforme à l'invention de composition suivante :

| | |
|---|---|
| - JAGUAR HP 60® | 1,7 g |
| - Ethanol | 20 g |
| - Eau déminéralisée qs | 100 g |

On applique 4 g de ce gel sur les racines des cheveux. On met en forme la chevelure à l'aide d'un peigne, puis on sèche les cheveux sous un casque. On obtient une chevelure disciplinée.
On a obtenu, avec cette composition, les mêmes propriétés cosmétiques qu'à l'exemple 1.

### EXEMPLE 3

On a préparé un autre gel conforme à l'invention de composition suivante :

| | |
|---|---|
| - JAGUAR HP 120® | 1 g |
| - Ethanol | 20 g |
| - Eau déminéralisée qs | 100 g |

On applique ce gel sur les cheveux. On met en forme la chevelure à l'aide d'un peigne ou des doigts, puis on sèche les cheveux avec un sèche-cheveux. On obtient une chevelure gonflante, le toucher est très naturel.
On a obtenu, avec cette composition, les mêmes propriétés cosmétiques qu'à l'exemple 1.

### EXAMPLE 4

On a préparé un gel conforme à l'invention de composition suivante :

| | |
|---|---|
| - Carboxyméthyl hydroxypropyl guar (GALACTASOL® 60 H 3 FD de AQUALON) | 1,7 g |
| - Ethanol | 17,2 g |
| - Acide citrique qs | pH 4 |
| - Eau déminéralisée qs | 100 g |

On a obtenu, avec cette composition, les mêmes résultats qu'à l'exemple 1.

### EXEMPLE 5

On a préparé un mascara conforme à l'invention de composition suivante :

| Phase A : | |
|---|---|
| - Cire d'abeille | 6,9 g |
| - Cire de carnauba | 4,16 g |
| - Paraffine | 11,4 g |
| - Acide stéarique | 7,7 g |

| Phase B : | |
|---|---|
| - Oxyde de fer noir | 5,55 g |

| Phase C: | |
|---|---|
| - Triéthanolamine | 3,8 g |
| - JAGUAR® HP 60 | 0,3 g |
| - Conservateurs qs | |
| - Eau déminéralisée qs | 100 g |

On fait fondre la phase A à 80°C puis on introduit la phase B que l'on disperse à l'aide d'un homogénéïsateur pendant 30 minutes.
On prépare la phase C en introduisant les trois premiers composants de cette phase dans l'eau portée à 75°C.
On réalise ensuite une émulsion en mélangeant la phase C à la phase A+B.

On a appliqué la composition de mascara sur les cils. Les cils présentent une bonne tenue.

## Revendications

1. Utilisation d'une gomme de guar non ionique ou anionique en tant que seul agent de mise en forme et/ou de maintien dans des compositions cosmétiques destinées à obtenir une mise en forme non permanente des fibres kératiniques.

2. Utilisation selon la revendication 1, caractérisée en ce que la quantité de gomme de guar est comprise entre 0,2 et 5% en poids par rapport au poids total de la composition.

3. Utilisation selon la revendication 2, caractérisée en ce que la quantité de gomme de guar est comprise entre 0,6 et 2% en poids par rapport au poids total de la composition.

4. Utilisation selon l'une quelconque des revendications 1 à 3, caractérisée par le fait que la gomme de guar non-ionique est modifiée par des groupements hydroxylakyle en C₁-C₆.

5. Utilisation selon la revendication 4, caractérisée par le fait que les groupements hydroxyalkyle sont choisis parmi les groupements hydroxyméthyle, hydroxyéthyle, hydroxypropyle et hydroxybutyle.

6. Utilisation selon l'une quelconque des revendications 1 à 5, caractérisée en ce que les gommes de guar non-ioniques sont modifiées par des groupements alkyle ou alkenyle, linéaires ou ramifiés, ayant de 8 à 30 atomes de carbone.

7. Utilisation selon l'une quelconque des revendications 1 à 3, caractérisée en ce que les gommes de guar non-ioniques sont modifiées par des groupements alkyle en C₁-C₆ et par des groupements alkyle ou alkenyle, linéaires ou ramifiés, ayant de 8 à 30 atomes de carbone.

8. Utilisation selon l'une quelconque des revendications 1 à 5, caractérisée par le fait que la gomme de guar non-ionique présente un taux d'hydroxyalkylation variant entre 0,4 et 1,2.

9. Utilisation selon l'une quelconque des revendications 1 à 3, caractérisée en ce que les gommes de guar anioniques sont modifiées par des groupements carboxyliques ou phosphate.

10. Utilisation selon la revendication 9, caractérisée en ce que les gommes de guar anioniques sont modifiées par des groupements carboxyméthyle.

11. Utilisation selon l'une quelconque des revendications 9 et 10, caractérisée en ce que les gommes de guar anioniques sont modifiées par des groupements hydroxyakyle en C₁-C₆.

12. Utilisation selon l'une quelconque des revendications précédentes, caractérisée en ce que lesdites compositions cosmétiques présentent un support aqueux ou hydroalcoolique.

13. Utilisation selon l'une quelconque des revendications précédentes, caractérisée en ce que lesdites compositions cosmétiques contiennent en outre un additif choisi parmi les conservateurs, les agents de régulation de pH, les parfums.

14. Utilisation selon l'une quelconque des revendications précédentes, caractérisée en ce que les fibres kératiniques sont des cheveux.

15. Utilisation selon l'une quelconque des revendications 1 à 10, caractérisée en ce que lesdites compositions cosmétiques sont des composition de fixation et/ou de coiffage.

16. Utilisation selon l'une quelconque des revendications 1 à 9, caractérisée en ce que les fibres kératiniques sont des cils ou des sourcils.

17. Utilisation selon l'une quelconque des revendications 1 à 13 et 16, caractérisée en ce que lesdites compositions cosmétiques sont des compositions de maquillage des cils ou des sourcils.

18. Procédé cosmétique de déformation et/ou de mise en forme non permanente des fibres kératiniques, caractérisé par le fait qu'il comprend les étapes suivantes :
(i) on applique sur les fibres kératiniques sèches ou humides une composition telle que définie à l'une quelconque des revendications précédentes,
(ii) on donne une forme aux fibres keratiniques à l'aide des doigts, d'un peigne ou d'une brosse,
(iii) on procède au séchage des fibres kératiniques ou on les laisse sécher,
(iv) éventuellement enfin, on coiffe les fibres kératiniques avec un peigne, une brosse ou avec les mains.

## Claims

1. Use of a nonionic or anionic guar gum as sole shaping and/or shape-retention agent in cosmetic compositions intended to obtain a temporary shaping of keratin fibres.

2. Use according to Claim 1, characterized in that the amount of guar gum is between 0.2 and 5 % by weight relative to the total weight of the composition.

3. Use according to Claim 2, characterized in that the amount of guar gum is between 0.6 and 2 % by weight relative to the total weight of the composition.

4. Use according to any one of Claims 1 to 3, characterized in that the nonionic guar gum is modified with C₁-C₆ hydroxyalkyl groups.

5. Use according to Claim 4, characterized in that the hydroxyalkyl groups are chosen from hydroxymethyl, hydroxyethyl, hydroxypropyl and hydroxybutyl groups.

6. Use according to any one of Claims 1 to 5, characterized in that the nonionic guar gums are modified with linear or branched alkyl or alkenyl groups having from 8 to 30 carbon atoms.

7. Use according to any one of Claims 1 to 3, characterized in that the nonionic guar gums are modified with C₁-C₆ alkyl groups and with linear or branched alkyl or alkenyl groups having from 8 to 30 carbon atoms.

8. Use according to any one of Claims 1 to 5, characterized in that the nonionic guar gum has a degree of hydroxyalkylation ranging between 0.4 and 1.2.

9. Use according to any one of Claims 1 to 3, characterized in that the anionic guar gums are modified with carboxylic or phosphate groups.

10. Use according to Claim 9, characterized in that the anionic guar gums are modified with carboxymethyl groups.

11. Use according to either of Claims 9 and 10, characterized in that the anionic guar gums are modified with C₁-C₆ hydroxyalkyl groups.

12. Use according to any one of the preceding claims, characterized in that the said cosmetic compositions have an aqueous or aqueous-alcoholic vehicle.

13. Use according to any one of the preceding claims, characterized in that the said cosmetic compositions additionally contain an additive chosen from preserving agents, pH regulators and fragrances.

14. Use according to any one of the preceding claims, characterized in that the keratin fibres are hair.

15. Use according to any one of Claims 1 to 10, characterized in that the said cosmetic compositions are hair styling and/or fixing compositions.

16. Use according to any one of Claims 1 to 9, characterized in that the keratin fibres are eyelashes or eyebrows.

17. Use according to any one of Claims 1 to 13 and 16, characterized in that the said cosmetic compositions are make-up compositions for the eyelashes or the eyebrows.

18. Cosmetic process for the temporary shaping and/or deformation of keratin fibres, characterized in that it comprises the following steps:
(i) a composition as defined in any one of the preceding claims is applied to the dry or wet keratin fibres,
(ii) the keratin fibres are shaped using the fingers, a comb or a brush,
(iii) the keratin fibres are dried or are left to dry,
(iv) finally, the keratin fibres are optionally styled with a comb, a brush or with the hands.

## Patentansprüche

1. Verwendung eines nichtionischen oder anionischen Guargummis als einzigen Wirkstoff zur Formung und/oder zum Formhalten in kosmetischen Zusammensetzungen, die dazu bestimmt sind, eine nicht permanente Verformung von Keratinfasern zu erreichen.

2. Verwendung nach Anspruch 1, dadurch gekennzeichnet, daß der Mengenanteil des Guargummis 0,2 bis 5 Gew.-%, bezogen auf das Gesamtgewicht der Zusammensetzung, beträgt.

3. Verwendung nach Anspruch 2, dadurch gekennzeichnet, daß der Mengenanteil des Guargummis 0,6 bis 2 Gew.-%, bezogen auf das Gesamtgewicht der Zusammensetzung, beträgt.

4. Verwendung nach einem der Ansprüche 1 bis 3, dadurch gekennzeichnet, daß das nichtionische Guargummi durch C₁-C₆-Hydroxyalkylgruppen modifiziert ist.

5. Verwendung nach Anspruch 4, dadurch gekennzeichnet, daß die Hydoxyalkylgruppen unter den Hydroxymethyl-, Hydroxyethyl-, Hydoxypropyl- und Hydroxybutylgruppen ausgewählt werden.

6. Verwendung nach einem der Ansprüche 1 bis 5, dadurch gekennzeichnet, daß die nichtionischen Guargummen durch geradkettige oder verzweigte Alkyl- oder Alkenylgruppen mit 8 bis 30 Kohlenstoffatomen modifiziert sind.

7. Verwendung nach einem der Ansprüche 1 bis 3, dadurch gekennzeichnet, daß die nichtionischen Guargummen durch C₁-C₆-Alkylgruppen und geradkettige oder verzweigte Alkyl- oder Alkenylgruppen mit 8 bis 30 Kohlenstoffatomen modifiziert sind.

8. Verwendung nach einem der Ansprüche 1 bis 5, dadurch gekennzeichnet, daß das nichtionische Guargummi einen Hydroxyalkylierungsgrad im Bereich von 0,4 bis 1,2 aufweist.

9. Verwendung nach einem der Ansprüche 1 bis 3, dadurch gekennzeichnet, daß die anionischen Guargummen durch Carboxyl- oder Phosphatgruppen modifiziert sind.

10. Verwendung nach Anspruch 9, dadurch gekennzeichnet, daß die anionischen Guargummen durch Carboxymethylgruppen modifiziert sind.

11. Verwendung nach einem der Ansprüche 9 und 10, dadurch gekennzeichnet, daß die anionischen Guargummen durch C₁-C₆-Hydroxyalkylgruppen modifiziert sind.

12. Verwendung nach einem der vorstehenden Ansprüche, dadurch gekennzeichnet, daß die kosmetischen Zusammensetzungen einen wäßrigen oder wäßrig-alkoholischen Träger aufweisen.

13. Verwendung nach einem der vorstehenden Ansprüche, dadurch gekennzeichnet, daß die kosmetischen Zusammensetzungen ferner einen Zusatz enthalten, der unter den Konservierungsstoffen, Mitteln zur Einstellung des pH-Werts und Parfums ausgewählt ist.

14. Verwendung nach einem der vorstehenden Ansprüche, dadurch gekennzeichnet, daß die Keratinfasern Haare sind.

15. Verwendung nach einem der Ansprüche 1 bis 10, dadurch gekennzeichnet, daß die kosmetischen Zusammensetzungen Festigungs- und/oder Frisierzusammensetzungen sind.

16. Verwendung nach einem der Ansprüche 1 bis 9, dadurch gekennzeichnet, daß die Keratinfasern Wimpern oder Augenbrauen sind.

17. Verwendung nach einem der Ansprüche 1 bis 13 und 16, dadurch gekennzeichnet, daß die kosmetischen Zusammensetzungen Zusammensetzungen zum Schminken der Wimpern oder Augenbrauen sind.

18. Kosmetisches Verfahren zur nicht dauerhaften Verformung und/oder Formgebung von Keratinfasern, dadurch gekennzeichnet, daß es folgende Schritte umfaßt:
(i) Auftragen einer Zusammensetzung nach einem der vorstehenden Ansprüche auf die trockenen oder feuchten Keratinfasern,
(ii) Formen der Keratinfasern mit den Fingern, einem Kamm oder einer Bürste,
(iii) Trocknen oder Trocknenlassen der Keratinfasern, und
(iv) gegebenenfalls Kämmen der Keratinfasern mit einem Kamm, einer Bürste oder mit den Händen.
